# EUROPEAN PATENT SPECIFICATION

(11) **EP 3 587 397 B1**
(45) Date of publication and mention of the grant of the patent: **02.03.2022**
(21) Application number: 18745278.4
(22) Date of filing: 25.01.2018
(51) Int. Cl.: A61K 31/197, A61P 25/28

(54) **S-ALLYL-L-CYSTEINE FOR USE IN THE TREATMENT OF MULTIPLE SCLEROSIS**
S-ALLYL-L-CYSTEIN ZUR VERWENDUNG IN DER BEHANDLUNG VON MULTIPLER SKLEROSIS
S-ALLYL-L-CYSTÉINE POUR L'UTILISATION DANS LE TRAITEMENT DE SCLÉROSE EN PLAQUE

(30) Priority: 26.01.2017 ES 201730093
(43) Date of publication of application: 01.01.2020
(73) Proprietor: Canvax Biotech, S.L., 14014 Córdoba (ES); Universidad De Córdoba, 14071 Córdoba (ES)
(72) Inventor: PAZ ROJAS, Elier, 14014 Córdoba (ES); GARCIA MACEIRA, Fe Isabel, 14014 Córdoba (ES); TORRES SANCHEZ, Luis Aristides, 14014 Córdoba (ES); RAMIREZ GONZALEZ, Vanesa, 14014 Córdoba (ES); GARCÍA RUBIÑO, María Eugenia, 14014 Córdoba (ES); TUNEZ FIÑANA, Isaac, 14071 Córdoba (ES); GIRALDO POLO, Ana Isabel, 14071 Córdoba (ES); ESCRIBANO DURÁN, Begoña María, 14071 Córdoba (ES); AGÜERA MORALES, Eduardo, Córdoba (ES); LUQUE CARABOT, Evelio, 14071 Córdoba (ES)
(74) Representative: Manuel Illescas y Asociados, S.L.
(86) International application number: PCT/ES2018/070056
(87) International publication number: WO 2018/138400

(56) References cited:
- EP-A1- 2 829 611
- EP-A2- 2 870 964
- WO-A2-2011/037411
- JP-A- 2015 144 571
- US-A- 5 206 270
- EL-MORSY IBRAHIM A.A.: "Evaluation of anti-proliferative and immunomodulatory effect of S-allyl cysteine on pulmonary fibrosis-induced rats", INTERNATIONAL JOURNAL OF PLANT, ANIMAL AND ENVIRONMENTAL SCIENCES, vol. 4, no. 2, 2014, pages 357-371, XP002798749,
- MIZUGUCHI SHINJIRO ET AL: "S-allyl cysteine attenuated CCl4-induced oxidative stress and pulmonary fibrosis in rats", BIOFACTORS, vol. 26, no. 1, 2006, pages 81-92, XP002798750, ISSN: 0951-6433
- TANG FENG-YAO ET AL: "Consumption of S-allylcysteine inhibits the growth of human non-small cell lung carcinoma in a mouse xenograft model", CANCER RESEARCH, vol. 71, no. Suppl. 8, April 2011 (2011-04), page 4605, XP002798751, & 102ND ANNUAL MEETING OF THE AMERICAN-ASSOCIATION-FOR-CANCER-RESEARCH (AACR); ORLANDO, FL, USA; APRIL 02 -06, 2011
- XU YA-SI ET AL: "S-allylcysteine, a garlic derivative, suppresses proliferation and induces apoptosis in human ovarian cancer cells in vitro", ACTA PHARMACOLOGICA SINICA, vol. 35, no. 2, February 2014 (2014-02), pages 267-274, XP002798752,
- CHU QINGJUN ET AL: "S-allylcysteine, a water-soluble garlic derivative, suppresses the growth of a human androgen-independent prostate cancer xenograft, CWR22R, under in vivo conditions", BJU INTERNATIONAL, vol. 99, no. 4, April 2007 (2007-04), pages 925-932, XP002798753,
- KIM KI-MO ET AL: "Differential regulation of no availability from macrophages and endothelial cells by the garlic component S-allyl cysteine", FREE RADICAL BIOLOGY AND MEDICINE, vol. 30, no. 7, 1 April 2001 (2001-04-01), pages 747-756, XP002798754, ISSN: 0891-5849
- COLÍN-GONZÁLEZ, A.L. ET AL.: 'On the antioxidant, neuroprotective and anti-inflammatory properties of S-allyl cysteine: An update' NEUROCHEMISTRY INTERNATIONAL, [Online] vol. 89, 2015, ISSN 0197-0186 pages 83 - 91, XP008177846 ISSN: 0197-0186 Retrieved from the Internet: <URL:http://dx.doi.org/10.1016/j.neuint. 2015-06.9 11> [retrieved on 2015-06-26]
- SHEN, C. ET AL.: 'In Vitro Biogeneration of Pure Thiosulfinates and Propanethial-S-oxide' JOURNAL OF AGRICULTURAL AND FOOD CHEMISTRY vol. 48, 2000, ISSN 0021-8561 pages 6254 - 6260, XP000992818
- FREEMAN, G.G. ET AL.: 'Synthetic S-Alk(en)yl-L-cysteine sulphoxides-alliinase fission products: Simulation of flavour components of Allium species' PHYTOCHEMISTRY vol. 15, 1976, pages 521 - 523, XP055528292
- DRABOWICZ, J. ET AL.: 'Reduction of sulfoxides. A review' ORGANIC PREPARATIONS AND PROCEDURES INTERNATIONAL: THE NEW JOURNAL FOR ORGANIC SYNTHESIS, [Online] vol. 16, 1984, pages 171 - 198, XP008022463 Retrieved from the Internet: <URL:http://dx.doi.org/1 0. 080/00304948409355456> [retrieved on 2009-02-10]
- ESCRIBANO, B.M. ET AL.: 'Dose-dependent S-allyl cysteine ameliorates multiple sclerosis disease-related pathology by reducing oxidative stress and biomarkers of dysbiosis in experimental autoimmune encephalomyelitis' EUROPEAN JOURNAL OF PHARMACOLOGY, [Online] vol. 815, 2018, ISSN 0014-2999 pages 266 - 273, XP085245767 ISSN: 0014-2999 Retrieved from the Internet: <URL:http://dx.doi.org/10.1016/j.ejphar. 2017.09.02 5> [retrieved on 2017-09-19]
- ZEINALI, H. ET AL.: 'S-allyl cysteine improves clinical and neuropathological features of experimental autoimmune encephalomyelitis in C57BL/6 mice' BIOMEDICINE AND PHARMACOTHERAPY, [Online] vol. 97, 2018, ISSN 0753-3322 pages 557 - 563, XP085323864 ISSN: 0753-3322 Retrieved from the Internet: <URL:http://dx.doi.org/10.1016/j.biopha. 2017.10.15 5>> [retrieved on 2017-11-06]
- DATABASE BIOSIS [Online] BIOSCIENCES INFORMATION SERVICE, PHILADELPHIA, PA, US; 2006, MIZUGUCHI SHINJIRO ET AL: "S-allyl cysteine attenuated CCl4-induced oxidative stress and pulmonary fibrosis in rats", Database accession no. PREV200600375964 & MIZUGUCHI SHINJIRO ET AL: "S-allyl cysteine attenuated CCl4-induced oxidative stress and pulmonary fibrosis in rats", BIOFACTORS, vol. 26, no. 1, 2006, pages 81-92, ISSN: 0951-6433(print)
- DATABASE BIOSIS [Online] BIOSCIENCES INFORMATION SERVICE, PHILADELPHIA, PA, US; April 2007 (2007-04), CHU QINGJUN ET AL: "S-allylcysteine, a water-soluble garlic derivative, suppresses the growth of a human androgen-independent prostate cancer xenograft, CWR22R, under in vivo conditions", Database accession no. PREV200700282387 & BJU INTERNATIONAL, vol. 99, no. 4, April 2007 (2007-04), pages 925-932, ISSN: 1464-4096(print), DOI: 10.1111/J.1464-410X.2006.06639.X
- DATABASE BIOSIS [Online] BIOSCIENCES INFORMATION SERVICE, PHILADELPHIA, PA, US; 1 April 2001 (2001-04-01), KIM KI-MO ET AL: "Differential regulation of no availability from macrophages and endothelial cells by the garlic component S-allyl cysteine", Database accession no. PREV200100205729 & KIM KI-MO ET AL: "Differential regulation of no availability from macrophages and endothelial cells by the garlic component S-allyl cysteine", FREE RADICAL BIOLOGY AND MEDICINE, vol. 30, no. 7, 1 April 2001 (2001-04-01), pages 747-756, ISSN: 0891-5849
- DATABASE BIOSIS [Online] BIOSCIENCES INFORMATION SERVICE, PHILADELPHIA, PA, US; April 2011 (2011-04), TANG FENG-YAO ET AL: "Consumption of S-allylcysteine inhibits the growth of human non-small cell lung carcinoma in a mouse xenograft model", Database accession no. PREV201600545839 & CANCER RESEARCH, vol. 71, no. Suppl. 8, April 2011 (2011-04), page 4605, 102ND ANNUAL MEETING OF THE AMERICAN-ASSOCIATION-FOR-CANCER-RESEARCH (AACR); ORLANDO, FL, USA; APRIL 02 -06, 2011 ISSN: 0008-5472(print), DOI: 10.1158/1538-7445.AM2011-4605
- DATABASE BIOSIS [Online] BIOSCIENCES INFORMATION SERVICE, PHILADELPHIA, PA, US; February 2014 (2014-02), XU YA-SI ET AL: "S-allylcysteine, a garlic derivative, suppresses proliferation and induces apoptosis in human ovarian cancer cells in vitro", Database accession no. PREV201400201847 & ACTA PHARMACOLOGICA SINICA, vol. 35, no. 2, February 2014 (2014-02), pages 267-274, ISSN: 1671-4083(print), DOI: 10.1038/APS.2013.176

## Description

### FIELD OF THE INVENTION

The present invention is defined in the appended claim. This disclosure relates to a process for obtaining extracts enriched in organosulphur compounds of types such as S-alkenyl-L-cysteine and S-alkyl-L-cysteine from *Allium* genus plants, free from sulphoxides, and to the use of the organosulphur compounds of types such as S-alkenyl-L-cysteine and S-alkyl-L-cysteine as therapeutic agents or food supplements for the prevention and treatment of human and animal diseases that incur in chronic inflammation.

### BACKGROUND OF THE INVENTION

Some plants that are attributed benefits in therapeutic applications belong to the *Allium* genus that includes approximately 500 species; the most used include garlic, onion, chives and leeks.

Garlic *(Allium sativum)* is one of the best-studied plants and has been used for centuries as a traditional remedy for a large number of health-related disorders. In addition, it is widely used as a food ingredient for its culinary appeal as a spice. From the clinical point of view, garlic has been evaluated for a number of therapeutic purposes, including: treatment of hypertension, hypercholesterolemia, diabetes, rheumatoid arthritis, the common cold, as well as the prevention of atherosclerosis and development of tumours, use as an antibiotic, anti-hypertensive and anti-thrombotic (Petrovska 2010). The therapeutic activity attributed to garlic is the result of a combination of biologically active metabolites present in the plant that act synergistically, notable among which are the organosulphur compounds and their precursors (Amagase 2006).

The main organosulphur compounds present in intact garlic are derivatives of cysteine where the hydrogen bonded to the sulphur atom of the cysteine has been replaced by an alkane group or by an alkene group, that is, a saturated or unsaturated aliphatic hydrocarbon. In turn these cysteines can be free or bound to a glutamyl group by the gamma position and, additionally, the sulphur atom can be reduced or oxidized as a sulphoxide group.

In relation to its wet weight, whole and intact garlic contains 1.8 % of sulphoxides of S-alkenyl-L-cysteine (for example, alliin) and sulphoxides of S-alkyl-L-cysteine (for example, methiin). In addition, it contains 0.9 % of other compounds of the gamma-glutamyl-S-alkenyl-L-cysteine type (for example, gamma-glutamyl-S-allyl cysteine and gamma-glutamyl-S-propionyl cysteine) and of the gamma-glutamyl-S-alkyl-L-cysteine type (such as gamma-glutamyl-S-methyl cysteine). These compounds represent almost 90 % of the total organosulphur compounds present in garlic.

The most recent experimental data attribute the beneficial effects of garlic mainly to the organosulphur compounds of the types S-alkenyl-L-cysteine and S-alkyl-L-cysteine, described as powerful antioxidants in commercial preparations of garlic, such as black garlic and aged garlic extract (AGE) (Colín-González, 2015). Intact garlic bulbs contain a small amount of S-alkenyl-L-cysteines and S-alkyl-L-cysteines, formed from the catabolism of their respective gamma-glutamyl-S-alkenyl-L-cysteines and gamma-glutamyl-S-alkyl-L-cysteines. The formation of these organosulphur compounds is catalysed by the action of the enzyme gamma-glutamyl transpeptidase (EC 2.3.2.2, γ-GTP) on the gamma-glutamyl-S-alkenyl-L-cysteines and the gamma-glutamyl-S-alkyl-cysteines present in the fresh garlic. However, in fresh garlic bulbs, this γ-GTP enzyme activity is very low and is limited to cell membranes, which hinders the production of antioxidant compounds.

When the garlic is triturated or crushed, the cells are disrupted and the enzyme alliinase or alliin lyase (EC 4.4.1.4) is released from the vacuole and catalyses the formation of thiosulfinates from the sulphoxides of S-alkenyl-cysteines and S-alkyl -L-cysteines present in cells. Thiosulfinates are involved in the defence of the plant due to its anti-microbial activity, but many studies have shown that compounds with potent antimicrobial activity have a negative impact on the gastrointestinal tract (Francino 2015). Notable among the thiosulfinates formed is allicin (diallyl thiosulfinate), responsible for the characteristic odour of garlic (Borlinghaus 2014). Thiosulfinates are highly unstable and decompose rapidly in a large variety of volatile sulphur compounds, such as diallyl sulphide, diallyl disulphide (diallyldisulphide), diallyl trisulphide (diallyltrisulphide), allyl methyl trisulphide, dithiins, vinyldithiins and ajoene. In addition to contributing to the characteristic smell of garlic, these compounds are responsible for causing bad breath, bad body odour and in high doses, or in particularly sensitive people, are responsible for many of the adverse reactions attributed to garlic, such as gastric irritation, allergic reactions and diarrhea (Amagase 2001).

It is necessary, therefore, to define a process for obtaining organosulphur compounds of type S-alkenyl-L-cysteine and S-alkyl-L-cysteine with high yield (high concentration of said compounds and to eliminate the undesirable compounds derived from the garlic as the sulphoxides of S-alkenyl-L-cysteines and sulphoxides of S-alkyl cysteine, to avoid their subsequent transformation into thiosulfinates). Once purified, these compounds can be used in therapeutic or nutraceutical compositions without the adverse effects mentioned above appearing in the individual. Prominent among the properties attributed to these compounds are their protective effect against oxidative stress, cancer, cardiovascular diseases, neurodegenerative diseases and hypocholesterolemic effect (Amagase 2006).

One of the most frequent forms of marketing garlic extracts rich in S-alkenyl-L-cysteines and S-alkyl-L-cysteines compounds is aged black garlic extract (AGE). AGE is obtained by incubating the garlic in 20 % aqueous ethanol for between 10 to 20 months at a temperature between 50 - 80 °C and controlled humidity between 70 - 95 %, after which the extract is filtered and concentrated. This ageing process results in a significant loss of thiosulfinates accompanied by an increase in the concentration of organosulphur compounds of type S-alkenyl-L-cysteine and S-alkyl-L-cysteine. During the ageing process of garlic, it is possible to increase the concentration of these compounds up to 5 times compared to fresh garlic. However, the biochemical mechanisms responsible for this increase are not completely defined (Bae 2014). In addition, the ageing process needs a period of between 30 days to 10 months to complete, so to maintain the proper temperature and humidity conditions throughout the process requires high energy consumption due to the required continuous energy supply.

It has been described that modifications of the garlic ageing process have managed to increase the concentration of organosulphur compounds such as S-allyl-L-cysteine (SAC) up to 8 times (Yamazaki 2008). The problem is that the final concentration of the compounds varies considerably in each preparation and depends on the processing method used. It is necessary, therefore, to define a process for obtaining uniform organosulphur compounds of the formulas S-alkenyl-L-cysteine and S-alkyl-L-cysteine for obtaining enriched extracts of these compounds and that can be used in clinical or biological tests in order to objectively determine their properties.

A method for increasing the concentration of SAC by eliminating allicin without involving an ageing process of fresh garlic has been described. Patent US 5,093,122 describes the addition of cysteine to an aqueous extract. The authors propose that the SAC compound is formed by the reaction of allicin with cysteine, thus enriching the extract in the compound of interest. However, although the concentration of SAC is increased by 22 times compared to fresh garlic, the use of cysteine leads to higher process costs and, in addition, the final residual cysteine can be toxic, as described (Klavins 1963; Yin; 2016). This process also does not eliminate the volatile compounds formed by the decomposition of allicin, responsible for the adverse effects indicated above.

It is, therefore, the object of the present disclosure to describe a process for obtaining plant extracts with a high concentration of organosulphur compounds of type S-alkenyl-L-cysteine and S-alkyl-L-cysteine from plants of the genus *Allium,* which lack the sulphoxides of S-alkenyl-L-cysteines and sulphoxides of S-alkyl-L-cysteine and their respective thiosulfinates. The procedure described here satisfactorily solves the aforementioned problem since it allows obtaining an extract with an enrichment of the compounds of interest by more than 50 times with respect to the plant starting material and with almost total elimination of the unwanted compounds. In addition, this procedure allows simplification in the process, decreasing the time and energy consumption.

The antioxidant effect of the compounds of type S-alkenyl-L-cysteines and S-alkyl-L-cysteines is due to the fact that these compounds can be used as a source of cysteine in the synthesis of glutathione (γ-glutamyl-L-cysteinylglycine), the main antioxidant of the cells. The relationship between reduced glutathione (GSH) and oxidized glutathione (GSSG) is the basis for redox homeostasis and is often used as an indicator of the cell's oxidative state and cellular toxicity. The presence of GSSG in high amounts or the deficiency of GSH reflects a state of oxidative stress, which translates into numerous pathologies.

The physiological availability of cysteine limits the formation of GSH, so that therapeutic strategies to combat oxidative stress have focused on the increase of cysteine levels as an indirect way for increasing GSH. Ingestion of oral cysteine as a supplement may promote the development of hypercysteinemia and potential poisoning, so other strategies are necessary. The main alternative for increasing the production of GSH is to use a precursor or an analogue of cysteine. The best-known substitute is N-acetylcysteine (NAC). The two main uses of NAC are as a mucolytic (Cantu-González 2014) and as an antidote to acetaminophen overdose (Heard 2008). The main known drawback of this compound is that it is a medicament that causes nausea, vomiting and dizziness and therefore cannot be taken for prolonged periods, which limits its use (Sandilands 2009).

In addition, contrary to what has been described, the authors of this document have observed that NAC has a pro-inflammatory effect, since after administration in animal models, the level of LBP (lipopolysaccharide binding protein) increases. LBP is a marker of the increase of LPS (bacterial lipopolysaccharide) in blood, one of the main markers of cellular inflammation. This surprising fact is a very important limitation for the continued use of NAC, so it should not be considered as a solution as a precursor of GSH and antioxidant.

Therefore, additionally, the present disclosure proposes the use as an antioxidant of organosulphur compounds of the formula S-alkenyl-L-cysteine and S-alkyl-L-cysteine, especially of S-allyl-C-cysteine, as therapeutic agents or food supplements for the prevention and treatment of human and animal diseases that incur in chronic inflammation.

### DESCRIPTION OF THE INVENTION

### Brief description of the invention

The present invention is defined in the appended claim. The present disclosure relates to a process for obtaining an extract of organosulphur compounds according to Formula 1 from plants of the genus *Allium,* characterized in that the extract obtained is free of sulphoxides, 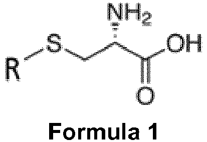 wherein the radical R is defined as an alkyl group or an alkenyl group, wherein the alkyl group corresponds to a straight or branched hydrocarbon chain, of from one to four fully saturated carbon atoms and the alkenyl group corresponds to a hydrocarbon chain, linear or branched, of from one to four carbon atoms, comprising at least one unsaturation and is not aromatic; characterized in that said method comprises the steps of:
i. inactivation of endogenous enzymes in the starting plant material;
ii. homogenization of the plant biomass;
iii. separation of the aqueous extract;
iv. gamma-deglutamylation of the aqueous extract;
v. removal of the sulphoxides present in the aqueous extract;
vi. purification;
vii. concentration;
viii. drying.

In one aspect of the disclosure, the extract obtained after step (iv) is subjected to a reduction step with reducing agents. In a more preferred embodiment, the reducing agent is selected from sodium hydrogen sulphite or sodium metabisulphite. In another aspect, the reduction is carried out in the presence of a catalyst. In another still more preferred embodiment, the catalyst used is molecular iodine.

In another aspect of the disclosure the process comprises an additional step after step (v) consisting of filtering the extract.

In another aspect of the disclosure the process described above comprises an additional step of crystallization or precipitation after step (vii).

In another aspect, the compounds of Formula 1 obtained by the process of the invention are selected from: S-allyl-L-cysteine (SAC), S-propyl-L-cysteine (SPC) and/or S-methyl-L-cysteine (SMC).

In another aspect, the starting plant material of the process of the invention is the bulb of a plant of the genus *Allium.*

In one aspect step (ii) of the process of the invention is performed by grinding the tissue in an aqueous medium.

In another aspect, step (iii) of the process of the invention is performed by filtration or by centrifugation.

In another aspect step (iv) of the process of the invention is carried out by incubating the extract obtained in step (iii) with an enzyme selected from the group consisting of: γ-glutamyl peptidase, γ-glutamyl transpeptidase and γ-glutamyl transferase.

In another aspect step (v) of the process of the invention is carried out by enzymatic treatment with alliinase.

In another aspect, step (vi) of the process of the invention is performed by ion exchange chromatography.

In another aspect, step (viii) of the process of the invention is carried out by lyophilisation, vacuum drying, air drying or by atomization.

In another aspect, the extract obtained by the process described above is enriched with the compounds of Formula 1 by more than 50 times with respect to the plant starting material. In another aspect, the extract is enriched with the compounds of Formula 1 by more than 70 times. In addition, said extract is stable and suitable for longterm storage and industrial application.

In another aspect, the amount of sulphoxides present in the extract is a percentage equal to or less than 20 % by mass with respect to the amount of S-alkenyl-L-cysteine and/or S-alkyl-L-cysteine present in the extract. In another aspect, the amount of sulphoxides present in the extract is a percentage equal to or less than 10 % by mass with respect to the amount of S-alkenyl-L-cysteine and/or S-alkyl-L-cysteine present in the extract.

The invention relates to a pharmaceutical or nutraceutical composition containing Z S-allyl-L-cysteine (SAC) for use in the treatment of multiple sclerosis.

### Description of the Figures

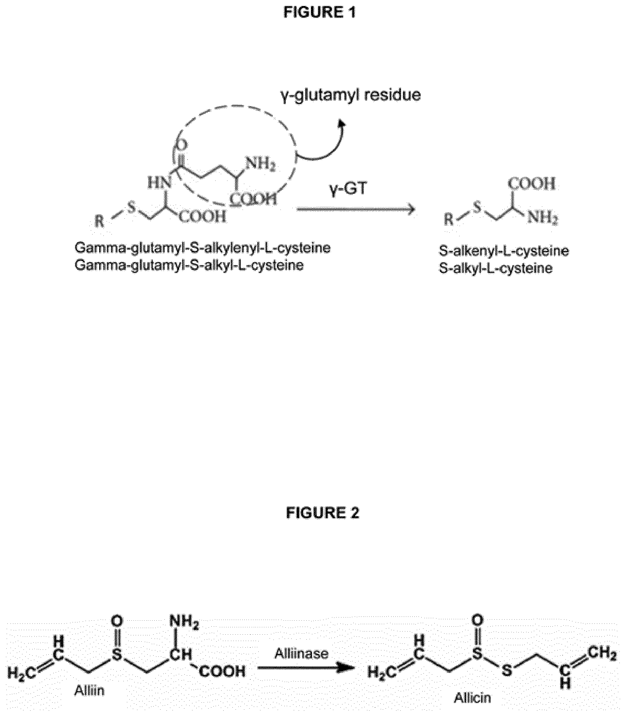

Figure 1: Scheme of the transformation of the compounds of type gamma-glutamyl-S-alkenyl-L-cysteine and gamma-glutamyl-S-alkyl-L-cysteine into S-alkenyl-L-cysteine and S-alkyl-L-cysteine compounds by treatment with the enzyme γ-glutamyl transferase (γ-GT). This process can be carried out by other enzymes such as γ-glutamyl transpeptidase (γ-GTP) or γ-glutamyl peptidase (γ-GP).
Figure 2: Scheme showing the transformation of alliin (S-allyl-L-cysteine sulphoxide) into allicin (its corresponding thiosulphate) mediated by the action of the enzyme alliinase.

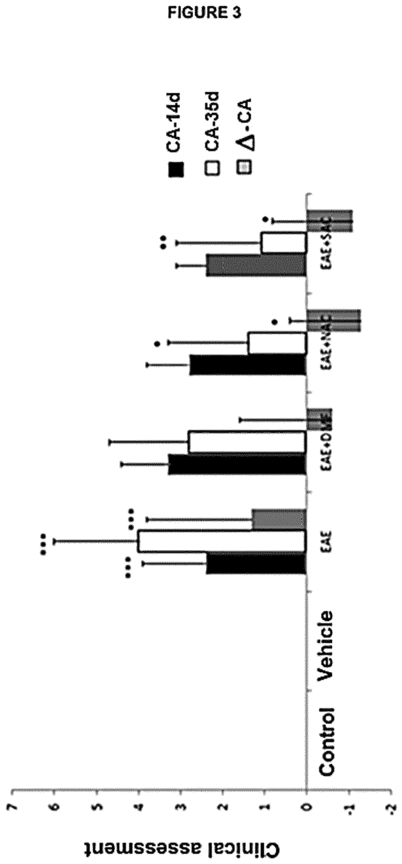

Figure 3: Effect of the treatments on the evolution of clinical mobility assessment of animals in which EAE (experimental autoimmune encephalomyelitis) has been induced. The treatments used are: DMF (dimethyl fumarate), NAC (N-acetyl cysteine) and SAC (S-allyl-L-cysteine), the vehicle (Freund's complete adjuvant, FCA) and the disease-free control. * *p* < 0.05 compared to control, *** *p* < 0.001 compared to control, ● *p* < 0.05 compared to EAE, •• *p* < 0.01 compared to EAE; ••• *p* < 0.001 compared to EAE. CA-14d (white bars): Clinical assessment of mobility at 14 days from the injection of MOG/FCA (MOG peptide with FCA, which induces the disease in animals) with each treatment (DMF, NAC or SAC); CA-35d (black bars): Clinical assessment of mobility at 35 days from the injection of MOG/FCA with each treatment (DMF, NAC or SAC) for 21 days; Δ-CA (grey bars): Change in the clinical assessment of mobility between day 35 (CA-35d) and day 14 (CA-14d). The clinical assessment represents the clinical assessment of the mobility of animals, which indicates greater disability as its value increases.

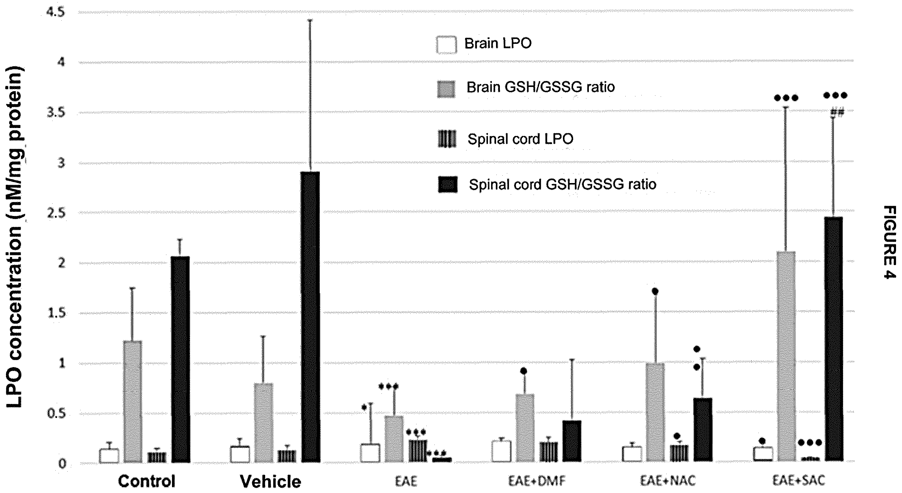

**Figure 4****:** Levels of biomarkers of oxidative stress at the end of the study (35 days). The injection of MOG/FCA to animals causes EAE, which is characterized by an increase in lipoperoxides (LPO) and a reduction in the GSH/GSSG ratio in both the spinal cord and the brain. The treatment with DMF, NAC and SAC induces an increase in the GSH/GSSG ratio although at different levels. **p*< 0.05 compared to control, ****p*< 0.001 compared to control, ●*p*< 0.05 compared to EAE, ●●*p*< 0.01 compared to EAE; ●●●*p*< 0.001 compared to EAE, ##p< 0.01 compared to EAE+NAC. The bars represent the mean ± standard deviation (SD). The clinical assessment represents the concentration of LPO expressed in nanomoles per milligram of protein (nM/mg protein).

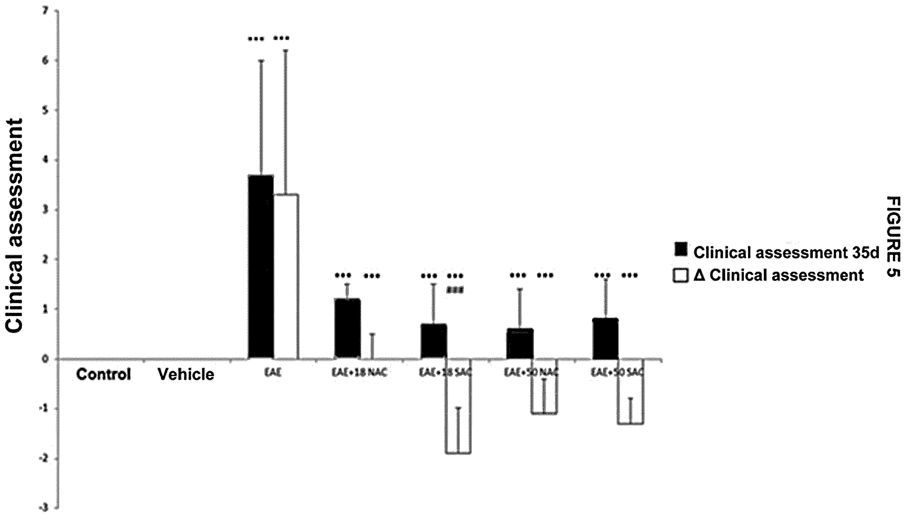

Figure 5: Effect on the clinical assessment of the mobility of treatments with NAC and SAC at different doses in animals with EAE induced by MOG/FCA. The change in the clinical assessment of mobility between day 35 and day 14 is indicated (Δ Clinical assessment, white bars) for each treatment (NAC and SAC) and dose 18 or 50 mg/kg. The bars represent the mean ± standard deviation (SD). *** *p* < 0.001 compared to the control group; ^{•••} *p* < 0.001 compared to the EAE group; *^{###} p* < 0.001 compared to EAE+18 NAC. The Clinical Assessment represents the clinical assessment of the mobility of animals, which indicates greater disability as its value increases.

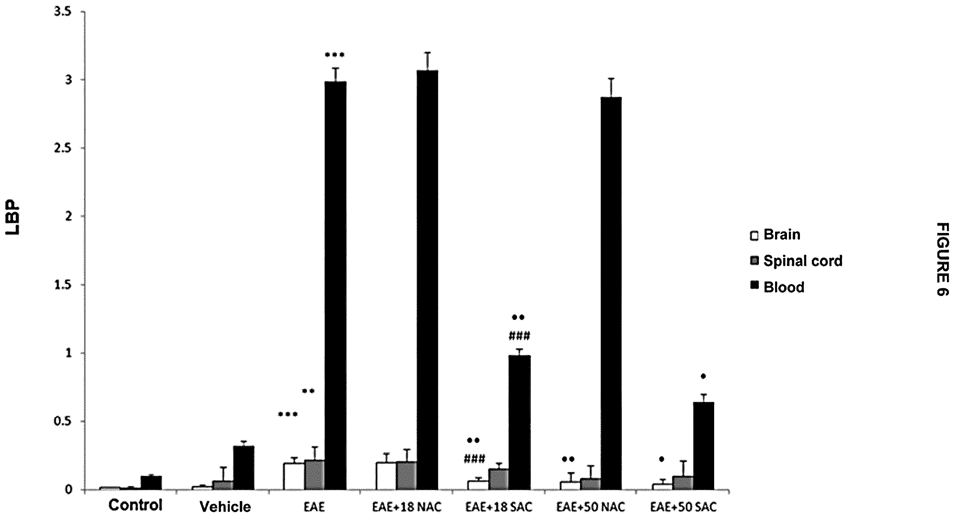

Figure 6: Change in the level of soluble LBP in different tissues and in blood due to the effect of NAC and SAC in animals with EAE induced by MOG/FCA. ****p* < 0.001 compared to control; ***p* < 0.01 compared to control; **p* < 0.05 compared to control; ●●●*p<* 0.001 compared to EAE; ●●*p* < 0.01 compared to EAE; ●*p* <0.05 compared to EAE; ###p <0.001 compared to EAE+NAC; ##p <0.01 compared to EAE+NAC. The clinical assessment represents the value of soluble LBP expressed as picograms per milligram of protein multiplied by 10,000 for the brain (white bars) and the spinal cord (grey bars), and in blood expressed as picograms of LBP per gram of haemoglobin multiplied by 10,000 (black bars). The values are represented as mean ± standard deviation (SD).

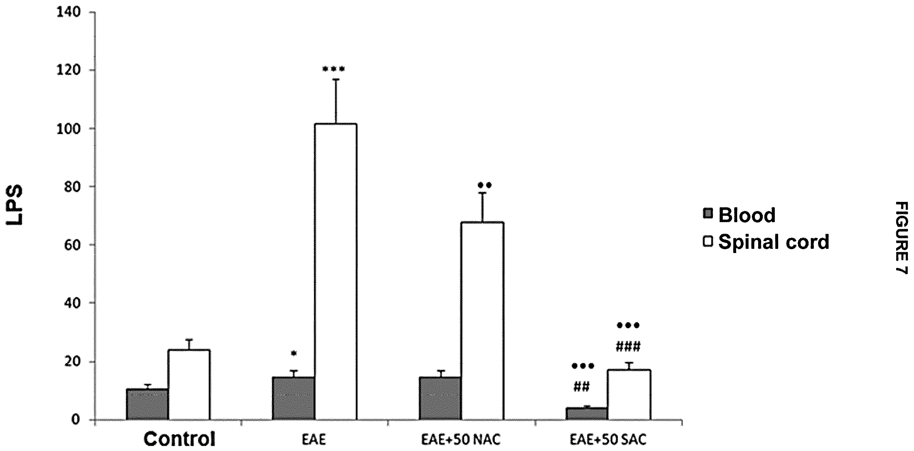

Figure 7: Change in the level of LPS in blood and spinal cord due to the effect of the treatment with NAC 50 mg/kg and SAC 50 mg/kg in animals with EAE induced by MOG/FCA. ****p*< 0.001 compared to Control; ***p*< 0.01 compared to Control; **p*< 0.05 compared to Control; ●●●*p*< 0.001 compared to EAE; ●●*p*< 0.01 compared to EAE; ●*p*<0.05 compared to EAE; ###p<0.001 compared to EAE+NAC; ##p<0.01 compared to EAE+NAC. The clinical assessment represents the concentration of LPS expressed as Units of Endotoxin per milligram of haemoglobin in blood (black bars) and as Units of Endotoxin per milligram of protein multiplied by 100 in the spinal cord (white bars). The values are represented as mean ± standard deviation (SD).

### Detailed description of the invention

The present disclosure describes a process for obtaining an extract rich in organosulphur compounds S-alkenyl-L-cysteine and S-alkyl-L-cysteine from plant raw materials and free from sulphoxides of S-alkenyl-L-cysteine and sulphoxides of S-alkyl-L-cysteine.

The organosulphur compounds S-alkenyl-L-cysteine and S-alkyl-L-cysteine according to the present disclosure are defined as compounds derived from the amino acid cysteine according to Formula 1, wherein the radical R- corresponds to an alkenyl group (compounds of formula S-alkenyl-L-cysteine) or to an alkyl group (compounds of formula S-alkyl-L-cysteine). In the present disclosure an alkyl group refers to a fully saturated straight or branched hydrocarbon chain, of from one to four carbons. An alkenyl group refers to a straight or branched hydrocarbon chain, of from one to four carbons that comprises at least one unsaturation and is non-aromatic.

In the present disclosure an extract free of sulphoxides of S-alkenyl-L-cysteine and sulphoxides of S-alkyl-L-cysteine is defined as a preparation containing sulphoxides of S-alkenyl-L-cysteine and sulphoxides of S-alkyl-L-cysteine in a percentage equal to or less than 30 %, preferably a percentage equal to or less than 20 % or more preferably equal to or less than 10 % by mass with respect to the amount of S-alkenyl-L-cysteine and S-alkyl-L-cysteine present in the extract.

In an aspect of the disclosure the organosulphur compounds of the type S-alkenyl-L-cysteine and S-alkyl-L-cysteine obtained according to the process of the invention, are the compounds S-allyl-L-cysteine (SAC, Formula 2), S-propyl-L-cysteine (SPC, Formula 3) and S-methyl-L-cysteine (SMC, Formula 4).

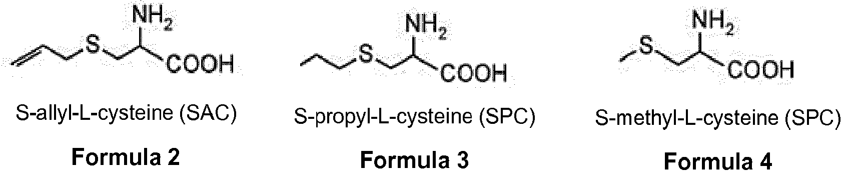

In another aspect of the disclosure the plant raw material from which the organosulphur compounds of the type S-alkenyl-L-cysteine and S-alkyl-L-cysteine defined by Formulas 1 to 4 are extracted is a plant of the *Allium* genus. In a particular aspect, the plants of the *Allium* genus are selected from the species *A. sativum, A. cepa, A. siculum, A. porrum, A. schoenoprasum, A. ascalonicum.* In another aspect, the organosulphur compounds of the types S-alkenyl-L-cysteine and S-alkyl-L-cysteine are obtained from the garlic plant *(Allium sativum).* Any part of the plant can be used in the process of the invention, although in a preferred embodiment the bulbs of said plants are used as the starting plant material. In a particular aspect of the disclosure, fresh bulbs recently harvested from *A. sativum* plants are used. In a still another aspect, fresh bulbs of the variety "purple garlic" of the species *A. sativum* are used.

In another aspect of the disclosure, the process for obtaining the organosulphur compounds of type S-alkenyl-L-cysteine and S-alkyl-L-cysteine from the plant raw materials mentioned above comprises the following steps:
i. inactivation of endogenous enzymes in the starting plant material;
ii. homogenization of the plant biomass;
iii. separation of the aqueous extract;
iv. gamma-deglutamylation of the aqueous extract;
v. removal of the sulphoxides present in the aqueous extract;
vi. purification;
vii. concentration;
viii. drying.

In step (i) of the process of the present disclosure, the starting plant material is subjected to an enzymatic inactivation. The inactivation conditions must be those in which the compounds of interest (compounds according to Formulas 1 to 4) do not undergo any chemical alteration. The main objective of this process is to stop all biochemical processes in the starting plant material, in particular, to inactivate the endogenous activity of the enzymes present in the bulbs of the plant. In this way, the organosulphur compounds of interest will remain unchanged in the starting material. In a preferred embodiment, the inactivation method is selected from wet heat and pH change. In a still more preferred embodiment, the enzyme inactivation method is wet heat. The wet heat by pre-cooking or blanching is the most widely known method and used in the food industry for the inactivation of enzymes and thus prevent their activity during the following steps of the process. In a particular embodiment of the invention, the starting plant material is submerged for 0.5 to 2 hours in hot water at a temperature of 60-110 °C. The plant material is submerged in water to a level where all the bulbs are covered by water, preventing their contact with the oxygen in the air. The temperature of the water should not be higher than 110 °C and the maximum time in which the starting plant material is submerged in the water must not be more than 2 hours. This step also causes the inactivation of the enzyme alliinase, so the sulphoxides of S-alkenyl-L-cysteine and S-alkyl-L-cysteine present from the start in fresh garlic remain in the extract, their conversion to thiosulfinates being prevented. Thiosulfinates are compounds that contain a functional group R-S(O)-S-R (where R- is an organic substituent).

In step (ii) of the process, the plant biomass of the previous step is homogenized. In one embodiment of the invention, the homogenization is performed by a mechanical process. In a more preferred embodiment, this second step is performed by trituration. The trituration is performed in an aqueous medium constituted by water or by an aqueous saline solution, optionally buffered at a pH from 3 to 10. The aqueous medium may contain one or more additives, such as detergents, flocculants, defoamers, etc.

In step (iii) of the process, the homogenized plant material is subjected to a process of separation of the solid biomass from the liquid phase to remove all solid waste and thus obtain an aqueous extract. In a preferred embodiment, the separation can be done by filtration or by centrifugation. The filtration is performed using, for example, but without limitation, filter press, band filters, rotary filters, press filters, etc., in which a barrier constituted by the filter support separates the plant biomass and allows the passage of the liquid extract without biomass. In a particular aspect of the disclosure, filtering is performed on a 100-hole per cm² nylon mesh screen and exerting sufficient pressure on the paste until the filtrate ceases to flow. Centrifugation makes use of the density differences between the aqueous extract and the biomass and, by means of a machine of the centrifugal separator type, decanter or similar, the solid phase is concentrated and separated from the liquid phase. The aqueous extract obtained in this step is formed by the soluble compounds of the plant starting material. The soluble organosulphur compounds of type S-alkenyl-L-cysteines, S-alkyl-L-cysteines, gamma-glutamyl-S-alkenyl-L-cysteines, gamma-glutamyl-S-alkyl-L-cysteines and their respective analogues are present in this aqueous extract, in addition to other soluble compounds such as sugars, proteins, polyphenols, etc.

Step (iv) of the process aims to enrich the aqueous extract of the organosulphur compounds of type S-alkenyl-L-cysteine and S-alkyl-L-cysteine. It consists in performing an enzymatic treatment that transforms the compounds of type gamma-glutamyl-S-alkenyl-L-cysteines and gamma-glutamyl-S-alkyl-L-cysteines to compounds of type S-alkenyl-L-cysteine and S-alkyl-L-cysteine respectively. In this step, the gamma-glutamyl residue of the previous substrates is cleaved by the action of an enzyme selected from the group comprising, without limitation, gamma-glutamyl peptidase (γ-GP), gamma-glutamyl transpeptidase (γ-GTP) and gamma-glutamyl transferase (γ-GT). The origin of the enzyme can be animal, plant or microbial. In a particular aspect, the enzyme used is gamma-glutamyl transferase (γ-GT). In another aspect of the disclosure, the origin of this enzyme is microbial, *Bacillus amyloliquefaciens* being the most preferred bacterium. In a particular aspect, the enzymatic treatment is performed for 2 to 16 hours at a temperature between 37 and 58 °C at pH between 5 and 8. As a final part of this step, the enzyme is inactivated by heat or by pH. In a particular embodiment of the invention, the enzyme is inactivated by heat at a temperature between 65 and 90 °C for between 15 minutes and 1 hour.

In one aspect of the disclosure, after step (iv), a step of reducing the sulphoxides of S-alkenyl-L-cysteine and the sulphoxides of S-alkyl-L-cysteine present in the aqueous extract takes place. The process for the reduction of sulphoxides requires the use of an easily accessible, manageable reducing agent that generates easily separated and environmentally safe by-products. Although the literature includes numerous reducing agents with the capacity to reduce sulphoxides to their corresponding thioether, many of these compounds need extremely strong reducing agents that also reduce other functional groups such as the carboxyl or alkene of the alliin, and therefore modify the final product that is obtained. Furthermore, many of these reactions give rise to by-products that are difficult to separate from the final product, so that tedious and expensive purification steps would be necessary to obtain the extract enriched with the compounds of interest. Many of the by-products, reducing agents described in the state of the art, are toxic and environmentally harmful (Drabowicz 1977). In one aspect of the disclosure, the reducing agent can be any compound capable of reducing all sulphoxides in the sample, such as, but not limited to: sodium metabisulphite (Na₂S₂O₅), sodium hydrogen sulphite (NaHSO3), NADPH⁺, CH₃COSH, Nal/HCI, DMS/HF and pinacol (2,3-dimethyl-2,3-butanediol). In a more preferred embodiment, the reducing agent is selected from Na₂S₂O₅ and NaHSO₃. In another aspect of the disclosure, the reducing agent is sodium hydrogen sulphite (NaHSO₃). In another aspect of the disclosure, the reducing agent acts in the presence of a catalyst. Any catalyst known to a person skilled in the art can be used, provided it is harmless and easily removable from the process. In a preferred embodiment, the catalyst of the reaction is molecular iodine (I₂). In a particular aspect of the disclosure, the extract obtained in step (iv) comes in contact with the reducing agent and the catalyst for 12 to 24 hours at a temperature between 25 and 90 °C and at a concentration between 10-20 %.

Step (v) of the process consists in the elimination of the sulphoxides of S-alkenyl-L-cysteine and the sulphoxides of S-alkyl-L-cysteine present in the aqueous extract. This step takes place by the enzymatic conversion of these compounds to their respective thiosulfinates. The thiosulfinates are unstable and decompose into volatile compounds that are completely removed in the following steps of the process. The enzyme that catalyses the reaction is a C-S lyase enzyme of microbial, plant or animal origin. In a particular aspect of the disclosure, the enzyme is Alliin lyase or Methionine γ-lyase. In another aspect, the enzyme used in this step is Alliin lyase (also known as Alliinase). In a preferred embodiment, the origin of the enzyme Alliinase is from plants. In another aspect of the disclosure, the enzyme is obtained from fresh garlic, in particular, *Allium sativum.* In a particular embodiment of the process, the reaction takes place at a temperature of 25-40 °C, at pH from 5 to 8 for 2-24 h. In a further aspect, the reaction takes place at 37 °C, pH 6 for 12 h.

In one aspect of the disclosure, after step (v), a filtration step of the obtained extract takes place. This step is optional and consists in the clarification by filtration of the extract obtained in step (v). The objective of this optional step is to remove the solid particles that may still be present in the extract. This optional step is carried out by means of a pressurised precoat filter, such as, for example, a frame and plate type filter, a leaf filter or a horizontal plate filter. The final passage diameter must be at least 1 µm. The solid particles removed in this step are discarded together with the filter and the obtained filtrate is subjected to the next step of the process.

Step (vi) of the process consists in the purification of the compounds of Formula 1. These compounds are non-proteinogenic sulphurated amino acids that can be purified using any conventional amino acid purification technique such as dialysis, ultrafiltration, chromatography, etc. In a preferred embodiment, the compounds of Formula 1 are purified by chromatography with a charged (H⁺) ion exchange column. In a further aspect, the ion exchange chromatography is carried out at a pH from 2 to 5, preferably at pH 5. The purification step (vi) consists in a first step in which the extract obtained in the previous step is incubated, with an ion exchange resin between 60 min and two hours, at room temperature, so that charged compounds such as amino acids present in the sample bind to the resin reversibly. Then, several 10-minute washes of the resin are carried out with water. In this way, the compounds present in the sample that are not bound to the resin are removed. Subsequently, the charged compounds bound to the resin are eluted with 2M NaOH for 1 hour and 30 minutes. Any other alkali with a pH between 8 and 14 can be used as an elution solution. The eluate obtained can optionally be desalted using any conventional known method, such as, for example, a reverse osmosis system or other suitable device. The thiosulfinates present in the sample are removed in this step because they are volatile and are not charged.

Once the eluate rich in organosulphur compounds of type S-alkenyl-L-cysteine and S-alkyl-L-cysteine (compounds of Formula 1) has been obtained, it is necessary to concentrate it (step vii). The concentration can be performed by any conventional method described in the state of the art, for example, by evaporation using a rotary evaporator in a thermostatic bath, using vacuum at a temperature between 40 °C and 70 °C depending on the vacuum pressure reached in the equipment or another suitable device.

In one aspect of the disclosure, the method described above optionally may include an additional step consisting in the precipitation or crystallisation of the eluate obtained in the above step (vii) by the addition of ethanol.

Finally, step (viii) consists in drying the extract obtained in the previous step. With drying, the preservation of the extract is sought by reducing the moisture content until it has the characteristics of a powder and thus extends its useful life. Examples of systems of this type are, but are not limited to, lyophilisation, vacuum drying, air drying, and atomization.

In one aspect of the disclosure, the purity of the compounds of type S-alkenyl-L-cysteine and S-alkyl-L-cysteine obtained by the method of the invention is measured by chromatography. In a further aspect, the purity of the organosulphur compounds present in the extract is measured by high-performance liquid chromatography (HPLC). In a further aspect, the purity of the organosulphur compounds of Formula 1 obtained by the method described above is higher than 60 %. In a preferred embodiment, the purity is higher than 70 %. In another aspect, the purity is higher than 80 %. In another aspect, the purity is higher than 90 %.

In another aspect, the extract obtained by the process described above is enriched with the compounds of Formula 1 by more than 50 times compared to the plant starting material. In a further aspect, the extract is enriched with the compounds of Formula 1 by more than 70 times compared to the plant starting material.

The present invention relates to a pharmaceutical or nutraceutical composition containing S-allyl-L-cysteine for use in the treatment of multiple sclerosis.

Chronic inflammation is considered to be inflammation of prolonged duration (weeks or months), in which signs of active inflammation, tissue damage and healing attempts can be simultaneously observed. Although it may be the evolution of symptoms of acute inflammation, chronic inflammation often begins as a low-intensity, often asymptomatic, hidden response. It is related to persistent infections caused by certain microorganisms, prolonged exposure to potentially toxic exogenous or endogenous agents and autoimmunity. It frequently occurs due to an exaggerated immune response, which causes damage instead of benefit in response to an infection. Examples of human and animal diseases that incur in chronic inflammation are: multiple sclerosis, rheumatoid arthritis, Alzheimer's, cancer, arteriosclerosis, ulcerative colitis, Crohn's disease, indeterminate colitis, collagenous colitis, lymphocytic colitis, tuberculosis, pulmonary fibrosis, lymphangitis, lymphadenitis, silicosis and bronchial asthma.

The organosulphur compounds of type S-alkenyl-L-cysteine and S-alkyl-L-cysteine have greater oral bioavailability and lack the intestinal mucolytic activity of other antioxidant compounds such as N-acetyl cysteine (NAC). The administration of NAC causes alterations on the mucosa that lead to their weakening, facilitating the translocation of LPS from the intestinal lumen and triggering a pro-inflammatory response in the organism. The absence of disulphide bridge reducing the activity of the organosulphur compounds of type S-alkenyl-L-cysteine and S-alkyl-L-cysteine makes it possible to explain their high bioavailability and the absence of the pro-inflammatory effect of NAC. The presence of the disulphide bridge reducing activity in NAC is the basis of its mucolytic activity, but it also seems to be responsible for its pro-inflammatory effect and its low bioavailability.

### EXAMPLES

The following examples are presented for the purpose of illustrating and describing in detail certain embodiments of the invention.

### Example 1: Obtaining an extract enriched in organosulphur compounds of type S-alkenyl-L-cysteine and S-alkyl-L-cysteine from garlic bulbs (Allium sativum)

As starting plant raw material, 5000 g of garlic bulbs *(Allium sativum)* of the variety purple garlic (ajo morado; La Veguilla SL) were used. The sample was subjected to a blanching treatment in water in which 3 volumes of water were added at a temperature of 80 °C for one hour. In this way, the endogenous enzymes of the starting plant material were inactivated. After this time, the sample was homogenized by trituration using an industrial blender (Sammic TR 250) at maximum speed according to the manufacturer's instructions.

The aqueous extract was separated from the solid biomass by filtration using a pressed layer filtration system (COLOMBO^{®}, Italy), with cellulose filters of 400, 200, 100 and 50 µm pore size arranged consecutively. To the obtained filtrate (aqueous extract), 0.5 U/ml of gamma-glutamyl transpeptidase (γ-GTP) from pork kidney was added and the extract was incubated at a pH of 6 and at a temperature of 37 °C for 12 hours. After incubation, the enzyme was inactivated by incubating at 80 °C for 20 minutes.

To remove the sulphoxides present in the extract, alliinase of vegetable origin, obtained from a crude extract of garlic bulbs (*A. sativum*) with C-S lyase activity, previously obtained by homogenizing fresh garlic in water in a ratio of 1 to 3 (1 gram of garlic for each 3 mL of water and separating the solid part of waste from the liquid extract). Next, this extract was incubated with the sample obtained in the previous step of the process for 8 hours at 37 °C and pH 6.

The obtained extract was purified by contacting it with a strongly acidic cation exchange matrix (Amberlite IR 120 H +) previously washed with water, at pH 4.75 for two hours. Then, two consecutive 10-minute washings with water were performed and the extract enriched with organosulphur compounds of type S-alkenyl-L-cysteine and S-alkyl-L-cysteine was eluted with 2M NaOH for 1 hour and 30 minutes, obtaining a fraction rich in the sulphurated amino acids of interest.

The fraction obtained in the previous step was concentrated with a rotavapor in a thermostatic bath (Rotavapor^{®} R-250 EX) at 60 °C and the product obtained was dried by lyophilisation (Telstar^{®} LyoBeta) obtaining 40 g of a powder composition.

To determine the amount of organosulphur compounds of type S-alkenyl-L-cysteine and S-alkyl-L-cysteine obtained by the above process, the amount of SAC present in the sample was analysed by HPLC (High-Performance Liquid Chromatography). Table 1 shows a comparison between the amount of SAC obtained, the amount of SAC in the starting material and the yield obtained from the aged garlic extract (AGE):

**Table 1**

| | S-allyl-L-cysteine (SAC) (mg/g) | Alliin (mg/g) |
|---|---|---|
| Fresh purple garlic bulbs (starting material) | 0.027 mg/g | 6.9 mg/g |
| AGE | 0.116 mg/g | 0.87 mg/g |
| Enriched extract ** | **6.84 mg/g** | 0.23 mg/g |

| | | |
|---|---|---|
| * AGE acquired from a commercial source ** Prepared according to the procedure described here | | |

As seen in Table 1, with the method of the invention, 59 times more SAC was obtained than with the methods described in the state of the art, such as the AGE production method. The process has also made it possible to greatly reduce the amount of alliin, which is found at a concentration of 7 mg/g in fresh garlic, while the method described here was able to reduce the amount of sulphoxides by approximately 30 times with respect to fresh garlic and by almost four times compared to the commercial AGE process. This process had a duration of approximately 24 hours, much shorter than the procedure used to obtain AGE (10-20 months).

### Example 2: Obtaining highly purified SAC using the method of the invention

1000 g of garlic shelled bulb *(Allium sativum)* of the variety purple garlic (ajo morado; La Veguilla SL) were used as starting plant raw material and were prepared and ground in the same conditions as in Example 1. Once the solid biomass was homogenised, it was separated from the aqueous extract, by filtration, using the same procedure of Example 1.

The liquid extract obtained was adjusted to pH 6.8 and then 0.25 U/ml of bacterial γ-glutamyl transferase (γ-GT) from *Bacillus amyloliquefaciens* was added and incubated at 55 °C for 4 hours. After incubation, the enzyme was inactivated by incubating the sample for 15 minutes at 90 °C.

The obtained aqueous extract was subjected to an enzymatic treatment with alliinase, purified and concentrated according to the procedure described in Example 1.

After concentrating the purified compound, the sample was precipitated with ethanol. For this, 4 volumes of 96 % ethanol were added in relation to the volume obtained in the concentration step. The organosulphur compounds present in the sample were precipitated and recovered by centrifugation at 6000 rpm for 10 minutes. Subsequently, the sample was crystallized by adding absolute ethanol until turbidity and then the sample was left for 24 hours under refrigeration (4 °C). After this time, the crystals were recovered by drying under vacuum at room temperature for 8 hours, obtaining 2.5 g of the final product.

The concentration and purity of SAC produced in this process were determined by HPLC (high-performance liquid chromatography). Table 2 shows a comparison between the amount of SAC obtained by the method described herein and the amount of SAC present in the starting material. Both quantities refer to the milligrams of SAC obtained per gram of fresh raw garlic in order to compare the procedures.

**Table 2**

| | S-allyl-L-cysteine (SAC) (mg/g) | Alliin (mg/g) | Purity |
|---|---|---|---|
| Fresh purple garlic bulbs (starting material) | 0.03 mg/g | 7.2 mg/g | 0.03 % |
| Enriched extract ** | 2.3 mg/g | 0.09 mg/g | 92 % |

| | | | |
|---|---|---|---|
| ** Prepared according to the procedure described here | | | |

As seen in Table 2, by means of the described procedure it is possible to obtain an extract of the S-allyl-L-cysteine compound (SAC) with a degree of purity above 90 %, and in addition the SAC/alliin ratio increased from 0.0042 in fresh garlic, up to 25.55 in the extract enriched by the method of the invention. This means that this procedure enriched the preparation of SAC compared to alliin by 6085 times, both increasing the amount of SAC produced per gram of garlic (increase by 76.6 times), and reducing the amount of alliin (80-fold decrease), the main sulphoxide present in garlic. The removal of sulphoxides using the methods described in the present invention makes it possible to obtain a product highly enriched in the main bioactive compound of garlic (SAC), which in turn has very low amounts of alliin, responsible for the decomposition products responsible for bad breath of garlic, gastric irritation caused by the consumption of garlic in many subjects and the destruction of part of the intestinal flora.

### Example 3: Use of the SAC obtained according to the methods of the present invention in the treatment of an animal model of multiple sclerosis (EAE), an autoimmune neurodegenerative disease

Next, an experiment was designed to study the effect of SAC in rats with EAE (experimental autoimmune encephalomyelitis), a model of multiple sclerosis widely used for the development of new treatments for this neurodegenerative disease in humans. To evaluate the effect of this compound on the disease, the change in clinical parameters of mobility in each of the joints and in the tail (clinical score or clinical assessment) was measured, as well as the biomarkers of oxidative stress that explain the change in joint mobility in rats.

70 Dark Agouti rats were used, 35 females and 35 males. Each group of 10 animals had 5 male rats housed in individual boxes and 5 females equally maintained in individual boxes. The following groups were included: i) Controls (untreated healthy animals); ii) vehicle (animals injected in the tail with 100 microlitres of Freund's complete adjuvant (FCA) but without the EAE-inducing peptide, the MOG peptide (oligodendrocyte myelin glycoprotein); iii) EAE (animals injected once into the tail with 150 µg of MOG (Sigma-Aldrich) in 100 microlitres of FCA); iv) EAE + dimethyl fumarate (EAE+DMF); v) EAE + N-acetyl cysteine (EAE+NAC); and vi) EAE + SAC. The treatments with NAC and SAC were administered to the animals orally using a probe and at a dose of 50 mg/kg of animal weight.

The mobility of the rats was evaluated using the method described by Perez-Nievas (Perez-Nievas, 2010) in which 0 is equivalent to not having any affected joint or tail; 1 equals paralysis in the tail; 2 equals partial mobility in the hind legs; 3 equals paralysis in the hind legs; 4 equals paralysis in the hind legs and partial mobility in the front legs and 5 equals a dying animal or that must be sacrificed for ethical reasons.

At the end of the study, the animals were decapitated at a controlled temperature using intraperitoneal anaesthesia with ketamine 75 mg/kg (Imalgene 100 mg/ml, Merial Laboratories, Barcelona, Spain). The brains, blood and spinal cord were extracted, weighed and homogenized with a mechanical homogenizer (Tempest Virtis), using 20 mM Tris at pH 7.4 as a buffer. The samples were centrifuged cold and the supernatants were immediately collected and stored at -80 °C.

Total protein levels were quantified by the Bradford method and referenced to the original tissue weight. Oxidative stress parameters such as lipid peroxidation (LPO), total glutathione (tG) and reduced glutathione (GSH) were then measured in blood, brain and spinal cord using kits from Bioxytech SA (Oxis International, Portland, OR, USA): LPO-586, GSH-420 and GSH-400, respectively. Oxidised glutathione levels (GSSG) were calculated by subtracting reduced glutathione from total glutathione and the GSH/GSSG ratio was calculated.

The statistical analysis was performed with the SPSS program (SPSS Ibérica, Madrid, Spain). The data are reported as mean ± standard deviation (SD). All the groups showed a normal distribution and therefore one-way ANOVA with Bonferroni correction was used. To analyse the correlation between the biochemical parameters and the mobility of the animals at 35 days (Clinical assessment-35), Spearman's rho correlation coefficient was used. The level of statistical significance was established at p <0.05.

The results showed a similar evolution between males and females in the clinical assessment of the disease as previously described in the state of the art, so no separate subgroups of males and females were analysed but the set of 10 animals together (**Figure 3**). Between days 14 and 35 in the group of animals in which the disease was induced using MOG in FCA and who did not receive treatment, there was an increase in the clinical assessment (CA) of mobility, that is, the animals showed a reduction of mobility in the legs and tail. The variation of the clinical assessment of the mobility obtained after subtracting the value at 14 days (CA-14d) from the value at 35 days (CA-35d) was positive in the animals with EAE and without treatment, which indicates that the mobility continued to decrease between days 14 and 35. The 3 groups of animals treated with DMF, NAC and SAC showed a reduction of the clinical assessment of mobility between day 35 and 14, that is, increased mobility as a consequence of treatment with DMF, which is a drug used in the clinic for the treatment of multiple sclerosis in humans. The same was observed with the treatment with N-acetyl-cysteine (NAC), a known antioxidant and with SAC (S-allyl-L-cysteine) derived from garlic obtained by the methods of the present invention. These results demonstrate that SAC can be an adequate food supplement for patients with multiple sclerosis.

However, given that these results seem to indicate that there are no differences between the use of NAC or SAC in the treatment of EAE, the different biomarkers of oxidative stress were analysed in order to verify if both treatments were really equivalent.

Oxidative damage was measured using the concentration of lipoperoxides (LPO) and the glutathione ratio between reduced glutathione (GSH) and oxidized glutathione (GSSG) in both the brain and the spinal cord. The injection of MOG in FCA (EAE) to the animals caused an increase in the levels of LPO, both in the brain and in the spinal cord, as well as a decrease in the GSH/GSSG ratio. As shown in **Figure 4**, treatments with DMF, NAC and SAC increased the GSH/GSSG ratio with respect to untreated animals (EAE), but it is also observed that there were statistically significant differences between the NAC and the SAC, with SAC being more effective than NAC in minimising oxidative damage induced by MOG/FCA.

In addition, possible correlations between the clinical assessment of mobility and biomarkers of oxidative stress were studied. A positive correlation was observed between the total glutathione level (Gt) and the lipoperoxide levels (LPO) in the brain (r = 0.406; p < 0.001), while there was a negative correlation in the brain between the clinical assessment of mobility on day 35 and the ratio between reduced glutathione (GSH) and oxidized glutathione (GSSG) (r = -0.237; p < 0.05) and between LPO and the GSH/GSSG ratio (r = -0.237; p < 0.01). In the spinal cord, a positive correlation was observed between the clinical assessment of mobility at 35 days and LPO levels (r = 0.559; p < 0.001). In addition, a negative correlation was observed in the spinal cord between the clinical assessment of clinical mobility on day 35 and the GSH/GSSG ratio (r = -0.586; p <0.001) and between the LPO levels and the GSH/GSSG ratio (r= -0.552; p < 0.001).

Taken together, these results indicate that the GSH/GSSG ratio and the LPO level are good biomarkers of oxidative damage and that SAC corrects the oxidative damage induced by MOG/FCA to a higher percentage than the NAC, which indicates that the SAC is a better supplement for patients with oxidative damage in the brain, such as that which occurs in patients with inflammatory diseases such as multiple sclerosis.

### Example 4: Comparison of clinical parameters of mobility and levels of LBP and LPS in animals with EAE induced with MOG/FCA and animals treated with NAC or SAC at different concentrations

Next, an experiment was designed to study whether the effect of NAC and SAC were different depending on the dose administered in animal models of EAE. For this, the change in clinical parameters of mobility in each of the joints and the tail (clinical score or clinical assessment) was measured at 2 different doses of NAC and SAC: 18 mg/kg and 50 mg/kg administered orally for 5 days a week and following the same experimental design as Example 3.

70 Dark Agouti rats, 35 females and 35 males, were used. Each group of 10 animals had 5 male rats housed in individual boxes and 5 females equally maintained in individual boxes. The following groups were included: i) Controls (untreated healthy animals); ii) vehicle (animals injected in the tail with 100 microlitres of Freund's complete adjuvant (FCA) but without the EAE-inducing peptide, the MOG peptide; iii) EAE (animals injected 1 time in the tail with 150 µg of MOG (Sigma-Aldrich) in 100 microlitres of FCA); iv) EAE + N-acetyl cysteine at 18 mg/kg (EAE + NAC18); v) EAE + N-acetyl cysteine at 50 mg/kg (EAE + NAC50); vi) EAE + SAC at 18 mg/kg (EAE + SAC18) and vii) EAE + SAC at 50 mg/kg (EAE + SAC50). The treatments with NAC and SAC were administered to the animals orally 5 days a week at a dose of 18 mg/kg or 50 mg/kg of the animal's weight. The analysis of the mobility of the animals, their sacrifice, tissue management, determination of the total proteins and the statistical analysis, was carried out as previously described in Example 3.

The measurement of lipopolysaccharide-binding soluble lipoprotein (LBP) levels was performed with the LBP soluble ELISA Kit (Enzo^{®}, USA), in a sandwich ELISA system following the manufacturer's instructions. The levels of lipopolysaccharide (LPS) in blood and spinal cord were measured with the Pierce LAL Chromogenic endotoxin quantification kit supplied by Thermo Scientific (USA), following the manufacturer's instructions.^{®} The data are presented as endotoxin units per milligram of tissue protein.

**Figure 5** represents the results of the change in the clinical assessment of mobility on day 35 as well as the increase between days 35 and 14 (Δ Clinical assessment). A reduction in the clinical assessment of mobility implies an increase in mobility as a result of the treatment. The dose of 18 mg/kg of NAC did not cause a reduction of the clinical assessment of mobility between days 35 and 14, which is interpreted as that there was no progression or improvement in the symptoms of the animals treated with NAC. Conversely, the use of 18 mg/kg of SAC caused a reduction in the clinical assessment of mobility equivalent to that caused by 50 mg/kg of NAC or SAC. This indicates that at a dose of 18 mg/kg of SAC was more effective than the equivalent dose of NAC. However, at a dose of 50 mg/kg, the differences between both treatments disappeared, which were equally effective with respect to the group of animals with EAE without treatment.

Then, the levels of soluble LBP protein in the brain, spinal cord and blood (Figure 6) and LPS in blood and spinal cord were measured (Figure 7). **Figure 6** shows how LBP increased as part of an acute phase response after the increase in LPS, which is one of the most potent proinflammatory agents known.

LPS is one of the most potent proinflammatory agents known. It is a component of the outer membrane of Gram-negative bacteria found in the intestinal flora. The intestinal wall is a barrier to the passage of bacterial products into the bloodstream and tissues, but can be made partially permeable to these products as a consequence of inflammatory conditions. In addition, there is a barrier to the passage of LPS formed by the mucus secreted by the intestinal wall. One of the widely known effects of NAC is its use as mucolytic, that is, an agent that breaks the mucus and makes it less viscous which promotes the secretion of the mucus. Despite NAC being a widely known antioxidant agent, its effect as mucolytic could reduce the thickness of the mucus layer and increase the permeability to intestinal LPS, which together with the pre-existing inflammation as a result of EAE induced by MOG/FCA could result in high levels of LBP and/or LPS in sick animals. If this is true, the beneficial effect of NAC as an antioxidant could be diminished by its mucolytic effect on the mucus of the membranes, especially in processes where NAC is administered chronically.

The results indicate that the development of EAE induced by injection of MOG/FCA in animals caused an increase of about 6 times the concentration of LBP in the blood, but also increased the amount of LBP in the brain and spinal cord with respect to untreated animals. This increase in soluble LBP is a consequence of the inflammatory reaction caused by EAE in animals. It should be expected that treatment with agents such as NAC and SAC would reduce inflammation and LBP levels. It was observed that the treatment with NAC at 18 mg/kg did not reduce the levels of LBP, either in blood or in the spinal cord or in the brain, whereas the NAC administered at 50 mg/kg reduced the levels of LBP only in the brain with respect to the untreated animals with EAE. However, SAC at 18 mg/kg caused a statistically significant reduction in the levels of LBP in both blood and brain compared to untreated animals with EAE and this same effect was observed at 50 mg/kg in both blood and brain. These results indicate that contrary to what would be expected by an expert, SAC behaves in a qualitatively as well as quantitatively different way to NAC, with the SAC being superior in the reduction of inflammatory biomarkers compared to NAC. When the LPS levels were measured, the same trend was observed as with LBP (**Figure 7**). It is important to point out that SAC at 50 mg/kg significantly reduced the levels of LPS in blood and spinal cord compared to NAC at the same concentration. This fact coincides with what had been obtained in the LBP levels.

Together, these results show that SAC is a better anti-inflammatory agent than NAC, since it reduces the levels of proinflammatory products such as LPS and LBP much more effectively, which increases after the increase in LPS. This fact, which is not evident to an expert, supports the use of SAC as support therapy in patients with inflammatory diseases where there is an oxidative imbalance as a better alternative than the use of NAC. This example in a neurodegenerative and inflammatory disease such as multiple sclerosis supports the use of SAC in the treatment of inflammatory diseases.

### BIBLIOGRAPHY

- Amagase H, Petesch B, Matsuura H, Kasuga S, Itakura Y. Intake of garlic and its bioactive components. J Nutr. 2001; 131: 955S-62S.
- Amagase H. Clarifying the real bioactive constituents of garlic. J Nutr. 2006; 136: 716S-725S.
- Bae SE, Cho SY, Won YD, Lee SH, Park HJ. Changes in S-allyl cysteine contents and physicochemical properties of black garlic during heat treatment. LWT-Food Sci and Technol. 2014; 55: 397-402.
- Borlinghaus J, Albrecht F, Gruhlke MCH, Nwachukwu ID, Slusarenko AJ. Allicin: Chemistry and Biological Properties. Molecules 2014; 19: 12591-12618.
- Cantu-Gonzalez G. 50 years ago in The Journal of Pediatrics: The use of N-acetylcysteine in the treatment of cystic fibrosis. J Pediatr. 2014 Oct; 165(4):721.
- Colín-González AL, Ali SF, Túnez I, Santamaría A. On the antioxidant, neuroprotective and anti-inflammatory properties of S-allyl cysteine: An update. Neurochemistry International. 2015; 89: 83-91.
- Drabowicz J, Numata T, Oae S. Dexygenation of sulfoxydes. A review. Organic Preparations and Procedures Int. 1977; 9(2): 63-83.
- Francino MP. Antibiotics and the Human Gut Microbiome: Dysbioses and Accumulation of Resistances. Frontiers in Microbiology 2015; 6: 1543.
- Heard KJ. Acetylcysteine for Acetaminophen Poisoning. The New England journal of medicine. 2008; 359(3):285-292.
- Klavins JV. Pathology of Amino Acid Excess: II. Effects of Administration of Excessive Amounts of Sulphur Containing Amino Acids: L-Cystine. British journal of experimental pathology 1963; 44(5): 516.
- Perez-Nievas BG, Garcia-Bueno B, Madrigal JL, Leza JC. Chronic immobilisation stress ameliorates clinical score and neuroinflammation in a MOG-induced EAE in Dark Agouti rats: mechanisms implicated. J Neuroinflammation 2010; 7, 60.
- Petrovska BB, Cekovska S. Extracts from the history and medical properties of garlic. Pharmacognosy Reviews 2010; 4(7): 106-110.
- Sandilands EA, Bateman DN. Adverse reactions associated with acetylcysteine. Clin Toxicol (Phila). 2009 Feb;47(2):81-8.
- Yamazaki Y, Okuno T. Accumulation of S-allyl-cysteine in garlic bulbs by warming. Nippon Shokuhin Kagatu Kogatu Kaishi 2008; 55(9):410-415.
- Yin J, Ren W, Yang G, Duan J, Huang X, Fang R, Li C, Li T, Yin Y, Hou Y, Kim SW, Wu G. L-Cysteine metabolism and its nutritional implications. Mol. Nutr. Food Res. 2016; 60: 134-146.
- US 5,093,122A. Method for preparing an S-allylcysteine-containing composition.

## Claims

1. Pharmaceutical or nutraceutical composition containing S-allyl-L-cysteine, for use in the treatment of multiple sclerosis, in humans or animals.

## Patentansprüche

1. Pharmazeutische oder nutrazeutische Zusammensetzung, die S-allyl-L-Cystein enthält, zur Verwendung bei der Behandlung von Multipler Sklerose bei Menschen oder Tieren.

## Revendications

1. Composition pharmaceutique ou nutriceutique contenant de la S-allyl-L-cystéine, pour l'utilisation dans le traitement de la sclérose en plaque, chez l'homme ou l'animal.
